# EUROPÄISCHE PATENTANMELDUNG

(11) **EP 3 973 873 A1**
(43) Veröffentlichungstag der Anmeldung: **30.03.2022**
(21) Anmeldenummer: 21196940.7
(22) Anmeldetag: 15.09.2021
(51) Int. Cl.: A61B 5/16

(54) **SYSTEM UND VERFAHREN ZUM ERFASSEN VON DATEN ZUR BEVORZUGUNG EINES HÖRERLEBNISSES BEI SÄUGLINGEN UND KLEINKINDERN**

(30) Priorität: 23.09.2020 DE 102020124842
(71) Anmelder: Universität Potsdam, 14469 Potsdam (DE)
(72) Erfinder: Boll-Avetisyan, Natalie, 10967 Berlin (DE)
(74) Vertreter: Dr. Klemens Schubert Patentanwalt

(57) **Zusammenfassung**

Offenbart wird ein System zum Erfassen von Daten zur Bevorzugung eines Hörerlebnisses bei Säuglingen und Kleinkindern, das mindestens ein Speichermedium zur Speicherung von Dateien, auf welchem mindestens zwei unterschiedliche akustische Dateien gespeichert sind, die sich darin voneinander unterscheiden, dass Kinder mit normaler Sprachentwicklung das Hörerlebnis einer der mindestens zwei akustischen Dateien bevorzugen, mindestens einen Lautsprecher, zur Wiedergabe der akustischen Dateien, mindestens zwei Bedienelemente, die selektiv das Abspielen der mindestens zwei akustischen Dateien initiieren, mindestens ein Detektionselement, welches die Anzahl und/oder Dauer der Betätigungen der mindestens zwei Bedienelemente detektiert, und mindestens ein Speicherelement, welches die vom mindestens einen Detektionselement detektierten Daten speichert, umfasst. Weiterhin wird ein Verfahren zum Erfassen von Daten zur Bevorzugung eines Hörerlebnisses bei Säuglingen und Kleinkindern offenbart, bei welchem die erfindungsgemäße Vorrichtung verwendet wird.

## Beschreibung

Die vorliegende Erfindung betrifft ein System und ein Verfahren zum Erfassen von Daten zur Bevorzugung eines Hörerlebnisses bei Säuglingen und Kleinkindern.

Sprachentwicklungsstörungen bei Kindern können verschiedene Ursachen haben. Gegenwärtig unterscheidet man begrifflich zwischen spezifischen und unspezifischen Sprachentwicklungsstörungen. Spezifische Sprachentwicklungsstörungen sind solche, die sich trotz guten Gehörs bei normaler Intelligenz und keinen weiteren physischen oder psychischen Auffälligkeiten des Kindes entwickeln. Von einer unspezifischen Sprachentwicklungsstörung wird momentan gesprochen, wenn daneben weitere Auffälligkeiten vorliegen wie beispielsweise Einschränkungen der Hörfähigkeit oder geistige Beeinträchtigungen. Abzugrenzen sind Sprachentwicklungsstörungen von sogenannten umgebungsbedingten Sprachauffälligkeiten, die durch Anregungsarmut oder im Rahmen eines mehrsprachigen Erwerbs verursacht sind und nicht klinisch relevant sind. Sprachentwicklungsstörungen hingegen haben eine hohe klinische Bedeutung, da die lediglich anregende Gestaltung ihrer sprachlichen Umgebung den betroffenen Kindern nicht hilft, ihre sprachlichen Probleme zu überwinden. Vielmehr ist eine störungsspezifische Therapie vonnöten.

Bislang können Sprachentwicklungsstörungen erst ab dem 4. Lebensjahr diagnostiziert werden. Sprachtherapeutische Interventionen starten dementsprechend spät, nämlich erst dann, wenn die wesentlichen Grundlagen der Sprache bereits erworben sein sollten. Betroffene Kinder bleiben häufig ihr Leben lang, auch trotz Therapie, sprachlich auffällig und erleiden in Folge oft psychische und schulische Probleme.

Die Sprachentwicklungsstörung ist in der Bevölkerung kaum bekannt, obwohl sie weit verbreitet und eine der häufigsten Entwicklungsauffälligkeiten ist. Circa sieben Prozent aller Kinder zeigen spezifische Sprachentwicklungsstörung, und vergleichbar viele Kinder zeigen unspezifische Sprachentwicklungsstörungen, die mit ähnlichen Auswirkungen verbunden sind. Die Symptome dieser Störung sind dramatisch. Im Einschulungsalter von sechs Jahren haben die betroffenen Kinder einen Rückstand ihrer sprachlichen Fähigkeiten von etwa ein bis vier Jahren. Diese Verzögerungen haben negative Folgen. Oft haben diese betroffenen Kinder Probleme, das Lesen und Schreiben zu lernen, und damit schlechtere Bildungschancen als nicht betroffene Kinder. Dies wirkt sich auf ihre späteren schulischen Leistungen aus und schränkt ihre Chancen in Bezug auf eine Berufsausbildung oder eine Hochschulausbildung ein.

Außerdem erfahren die betroffenen Kinder häufig eine Ausgrenzung durch ihre Altersgenossen, und haben damit einhergehend ein höheres Risiko psychisch zu erkranken und häufig Schwierigkeiten, soziale Bindungen einzugehen.

Gegenwärtig wird die Sprachentwicklungsstörung häufig zu spät erkannt, um frühzeitig therapeutische Maßnahmen zu ergreifen. Zwar können früh einsetzende Sprachtherapien eine Sprachentwicklungsstörung nicht heilen, jedoch können diese Therapien die Symptome lindern und damit negative Auswirkungen auf die Betroffenen einschränken. Des Weiteren können sie verhindern, dass die Schere zwischen gestörtem und ungestörtem Spracherwerb mit zunehmendem Alter der Kinder immer weiter auseinander klappt.

Im Idealfall begänne man möglichst früh mit therapeutischen Interventionen. Dies setzt allerdings voraus, dass auch frühzeitig die Diagnose getroffen wird. Derzeit ist eine Diagnose allerdings erst ab dem vierten Lebensjahr möglich. Momentan wird bereits im Rahmen der kinderärztlichen U7-Untersuchung (um den zweiten Geburtstag des Kindes) erfasst, ob ein Kind weniger als 50 Wörter sprechen kann und damit ein Risiko für eine Sprachentwicklungsstörung besteht. Da aber etwa die Hälfte der Kinder, bei denen dieses Risiko zu bestehen scheint, ihren Rückstand bis zum dritten Geburtstag aufholen, wird bis zur tatsächlichen Diagnose auf eine Therapie verzichtet. Währenddessen entsteht bei den Kindern, die den Rückstand nicht aufholen, ein immer größerer sprachlicher Abstand zu den sich ungestört entwickelnden gleichaltrigen Kindern, der kaum mehr aufgeholt werden kann.

Um frühzeitig therapeutisch eingreifen zu können, muss diese Störung auch frühzeitig erkannt werden. Aktuell weiß die Forschung, dass die ersten Anzeichen einer Sprachentwicklungsstörung bereits im Babyalter präsent sind. Kinder, die sich später sprachlich auffällig entwickeln, zeigen bereits ab einem Alter von vier Monaten erste Auffälligkeiten in der Sprachwahrnehmung. Man weiß zum Beispiel, dass Babys früh auf Sprachmelodien reagieren, und dass das Erkennen von Sprachmelodien ein wichtiger Wegbereiter für den Spracherwerb ist. Kinder mit einer Sprachentwicklungsstörung haben hiermit Schwierigkeiten, die sich - wie Langzeitstudien zeigen - bereits im Babyalter ankündigen.

Die klassische Methoden der Spracherwerbsforschung erfordern üblicherweise komplexe Versuchsaufbauten, bei denen beispielsweise Blickbewegungs- und Hirnstrommessungen durchgeführt werden. Diese Methoden können jedoch nicht diagnostisch eingesetzt werden. Sie sind ausschließlich für Gruppenstudien ausgelegt. Daten einzelner Kinder, die mittels dieser Verfahren ermittelt werden, sind nicht aussagekräftig.
Zur Anwendung dieser Methoden muss der Säugling oder das Kleinkind in dem entsprechenden Setting anwesend sein, eine Durchführung des Verfahrens im Lebensumfeld des Kindes ist nicht möglich.

Auch sind diese komplexen Versuchsaufbauten kostenintensiv und an eine technische Infrastruktur gebunden. Die Durchführung der Verfahren an Orten mit einem geringen technischen Standard und geringen finanziellen Mitteln, wie zum Beispiel in Entwicklungsländern, ist schwerlich realisierbar.
Somit stehen komplexe Verfahren und Vorrichtungen nur einem begrenzten Patientenkreis zur Verfügung.

Ein Beispiel einer solchen komplexen Vorrichtung und eines damit ausführbaren Verfahrens offenbart das europäische Patent EP 2 114 240 B1. Offenbart werden Verfahren und Systeme zur Untersuchung von jungen Säuglingen mit Defiziten in der Hörverarbeitung. Diese Untersuchung kann auch nicht im Lebensumfeld des Säuglings oder Kleinkinds erfolgen, sondern muss an dem Ort durchgeführt werden, wo die Vorrichtung aufgebaut ist und Mitarbeiter zur Bedienung der Vorrichtung anwesend sind.

Aufgabe der vorliegenden Erfindung ist es daher, die Nachteile des Standes der Technik zu überwinden und ein System und ein Verfahren bereitzustellen, womit bereits bei Säuglingen und Kleinkindern Daten zur Bevorzugung eines Hörerlebnisses erfasst werden können. Hierbei ist es insbesondere die Aufgabe, eine wenig komplexe Vorrichtung bereitzustellen und das Verfahren in Bezug auf die Ermittlung von Daten im Lebensumfeld des Säuglings oder Kleinkinds durchzuführen, ohne dass die Vorrichtung die Anwesenheit von Bedienpersonal erfordert.

Die mittels der erfindungsgemäßen Vorrichtung und des erfindungsgemäßen Verfahrens ermittelten Daten zur Bevorzugung eines Hörerlebnisses können bei der Früherkennung von Sprachentwicklungsstörungen bei Säuglingen und Kleinkindern genutzt werden. Die Daten können aber auch allgemein in der frühkindlichen Entwicklungsforschung für die Erforschung der Entwicklung von Präferenzen des Hörerlebnisses bei sowohl sich typisch als auch atypisch entwickelnden Kindern eingesetzt werden. In diesem Forschungskontext können das erfindungsgemäße System und das erfindungsgemäße Verfahren sowohl bei Gruppenstudien als auch bei Studien individueller Kinder eingesetzt werden.

Die Aufgabe der Erfindung wird gelöst durch die Bereitstellung des erfindungsgemäßen Systems und des erfindungsgemäße Verfahrens, das mittels des erfindungsgemäßen Systems durchgeführt wird.

Das erfindungsgemäße System zum Erfassen von Daten zur Bevorzugung eines Hörerlebnisses bei Säuglingen und Kleinkindern ist kostengünstig herstellbar und das erfindungsgemäße Verfahren kann im Lebensumfeld eines Säuglings oder Kleinkinds ausgeführt werden.

Die Aufgabe der Erfindung wird also gelöst durch die Bereitstellung des erfindungsgemäßen Systems und des erfindungsgemäßen Verfahrens zum Erfassen von Daten zur Bevorzugung eines Hörerlebnisses.

Das erfindungsgemäße System zum Erfassen von Daten zur Bevorzugung eines Hörerlebnisses umfasst mindestens ein Speichermedium zur Speicherung von Dateien, auf welchem mindestens zwei unterschiedliche akustische Dateien gespeichert sind, die sich darin voneinander unterscheiden, dass Kinder mit normaler Sprachentwicklung das Hörerlebnis einer der mindestens zwei akustischen Dateien bevorzugen, mindestens einen Lautsprecher, zur Wiedergabe der akustischen Dateien, mindestens zwei Bedienelemente, die selektiv das Abspielen der mindestens zwei akustischen Dateien initiieren, mindestens ein Detektionselement, welches die Anzahl und/oder Dauer der Betätigungen der mindestens zwei Bedienelemente detektiert, und mindestens ein Speicherelement, welches die vom mindestens einen Detektionselement detektierten Daten speichert.

Erfindungsgemäß bevorzugt ist das erfindungsgemäße System, bei welchem die mindestens zwei verschiedenen akustischen Dateien Sprachdateien oder nichtsprachliche Ton-Dateien sind, die ausgewählt sind aus Sprachdateien mit unterschiedlichem Betonungsmuster, Sprachdateien mit unterschiedlichem Satzintonationsmuster, Sprachdateien mit unterschiedlichem Sprachrhythmus, Sprachdateien mit unterschiedlichen Sprachlauten, Sprachdateien mit unterschiedlichen Phonemen, Sprachdateien mit für das Kind muttersprachlicher oder nicht muttersprachlicher Sprache, Sprachdateien mit für das Kind nicht muttersprachlicher Sprache, Sprachdateien mit kindgerechter Sprache, Sprachdateien mit nicht kindgerechter Sprache, Geräuschen, nichtsprachlichen Tonabfolgen und Melodien.

Bevorzugt ist weiterhin, dass das erfindungsgemäße System mindestens eine Rechnereinheit mit Software umfasst.

Weiterhin bevorzugt ist das erfindungsgemäße System, bei welchem die mindestens eine Rechnereinheit mit Software und/oder das mindestens eine Speicherelement Komponenten des Detektionselements sind.

Vorteilhaft ist es, dass das erfindungsgemäße System mindestens eine Vorrichtung umfasst, in welcher Elemente des Systems gemeinsam in einem Gehäuse aufgenommen sind.

Erfindungsgemäß bevorzugt ist es, dass das erfindungsgemäße System eine Vorrichtungen umfasst, in welcher ein Speichermedium, zur Speicherung mindestens einer akustischen Datei, ein Lautsprecher und ein Detektionselement gemeinsam in einem Gehäuse aufgenommen sind, an welchem zwei Bedienelement angeordnet sind.

Insbesondere vorteilhaft ist, dass das erfindungsgemäße System zwei separate Vorrichtungen umfasst, in welchen jeweils ein Speichermedium, zur Speicherung mindestens einer akustischen Datei, ein Lautsprecher und ein Detektionselement gemeinsam in einem Gehäuse aufgenommen sind und an jedem der beiden Gehäuse ein Bedienelement angeordnet ist.

Weiterhin bevorzugt ist das erfindungsgemäße System, bei welchem die mindestens eine Vorrichtung jeweils mindestens eine Rechnereinheit mit Software umfasst.

Insbesondere vorteilhaft ist das erfindungsgemäße System, bei welchem die mindestens zwei akustischen Dateien Sprachdateien sind, die mit unterschiedlicher Sprachmelodie gesprochener, identischer Text sind, und/oder die mindestens zwei Sprachdateien von demselben Sprecher gesprochen wurden.

Weiterhin vorteilhaft ist das erfindungsgemäße System, bei welchem das mindestens eine Speichermedium zur Speicherung von Sprachdateien ein USB-Stick, eine SD-Speicherkarte oder eine Festplatte ist, und/oder die mindestens zwei Bedienelemente ausgewählt sind aus mechanischen Knöpfen, mechanischen Hebeln, mechanischen Tasten und Berührungssensoren, und/oder das mindestens eine Detektionselement ausgewählt ist aus mechanischen, elektronischen und softwaregestützten Detektionselementen, und/oder das mindestens eine Detektionselement ein Speicherelement umfasst, welches ein USB-Stick, eine SD-Speicherkarte oder eine Festplatte ist.

Die Aufgabe der Erfindung wird weiterhin gelöst durch das erfindungsgemäße Verfahren zum Erfassen von Daten zur Bevorzugung eines Hörerlebnisses bei Säuglingen und Kleinkindern, a) wobei man das erfindungsgemäße System bereitstellt, b) man dem Säugling oder Kleinkind mindestens einen Zeitraum lang den Zugriff auf die Bedienelemente des Systems ermöglicht, c) man detektiert, wie oft und/oder wie lange der Säugling oder das Kleinkind die mindestens zwei Bedienelemente des Systems in dem mindestens einen Zeitraum betätigt, d) man basierend auf der Anzahl und/oder Dauer der Betätigungen der mindestens zwei Bedienelemente des Systems beim Säugling oder Kleinkind eine Bevorzugung einer der akustischen Dateien ermittelt.

Bevorzugt ist weiterhin das erfindungsgemäße Verfahren, bei welchem man das Verfahren im natürlichen Lebensumfeld des Säuglings oder Kleinkinds durchführt.

Erfindungsgemäß vorteilhaft ist das erfindungsgemäße Verfahren, bei welchem der mindestens eine Zeitraum in Schritt b) zwischen 1 und 20 Minuten beträgt.

Weiterhin bevorzugt ist das erfindungsgemäße Verfahren, bei welchem man Schritt b) über mehrere Zeiträume durchführt.

Untersuchungen haben gezeigt, dass Säuglinge und Kleinkinder mit altersgemäßer Sprachentwicklung typischerweise zu bestimmten Alterszeitpunkten beispielsweise zwischen bestimmten Sprachmelodien und andere Eigenschaften von gesprochener Sprache unterscheiden können. Auch können Kinder bereits im Säuglings- und Kleinkindalter zum Beispiel zwischen nichtsprachlichen Tönen und Tonabfolgen (beispielsweise Musik oder andere Geräusche) differenzieren. Diese Unterscheidung führt üblicherweise dazu, dass diese Kinder eine Präferenz für das ihnen bekannte Hörerlebnis zeigen oder für das Hörerlebnis, das einem ihnen bekannten ähnlicher ist. Manchmal zeigen die Kinder jedoch auch eine umgekehrte Präferenz und bevorzugen das neue, unbekanntere Ton- oder Sprachsignal. Es wird davon ausgegangen, dass die untersuchten Säuglinge und Kleinkinder mittels dieser Präferenz entweder zeigen, was sie aus ihrer Umwelt als Bekanntes und für sie natürliches Hörerlebnis wahrgenommen und somit erlernt haben. Oder sie zeigen mittels dieser Präferenz ein universales, möglicherweise angeborenes Interesse für bestimmte Hörerlebnisse.

Ergeben die ermittelten Daten, dass keine oder nur eine schwache Bevorzugung für eine akustische Datei festgestellt werden kann, können der Säugling oder das Kleinkind der Risikogruppe für das Bestehen einer Sprachentwicklungsstörung zuordnet werden.

In dieser Anmeldung werden die von einem Kind mit normaler Sprachentwicklung bevorzugten akustischen Dateien, die auf dem Bekannten und Erlernten oder universal Präferierten basieren, auch als natürliche akustische Dateien bezeichnet.

Liegt eine Sprachentwicklungsstörung bei einem Säugling oder Kleinkind vor, so zeigt dieses Kind nicht die oben beschriebene Präferenz, oder nur eine schwache Präferenz, und bevorzugt damit nicht das Hörerlebnis im selben Maße wie ein Kind ohne Sprachentwicklungsstörung.

Stellt man den Säuglingen und Kleinkindern die Möglichkeit bereit, zwischen dem Hörerlebnis von mindestens zwei akustischen Dateien zu wählen, also beispielsweise zwischen zwei Sprachdateien, in natürlicher und nicht natürlicher Sprachmelodie gesprochen, wählen die Kinder mit normaler Sprachentwicklung bevorzugt die Sprachdatei mit natürlicher Sprachmelodie. Kinder mit einer Sprachentwicklungsstörung zeigen diese Präferenz nicht oder deutlich weniger ausgeprägt als sich typisch entwickelnde Kinder.

Da bereits Säuglinge und Kleinkinder imstande sind, altersgerechte Spielzeuge zu betätigen, können sie die erfindungsgemäße Vorrichtung - bei Bedarf nach einer optionalen Lernphase - betätigen und die gespeicherten akustischen Dateien selektiv abspielen.

Je nachdem, wie lange und/oder wie häufig ein Bedienelement zum Abspielen einer der akustischen Dateien vom Kind betätigt wurde, wird abgeleitet, ob eine Präferenz für eine bestimmte akustische Datei beim Kind vorliegt. Liegt keine Präferenz oder eine nur sehr schwache Präferenz vor, oder geht die Präferenz in die unerwartete, untypische Richtung (wenn beispielsweise Sprache mit nicht natürlicher Sprachmelodie bevorzugt wird), ist dies unter Umständen als Hinweis auf eine vorliegende Sprachentwicklungsstörung zu werten.

Die Bereitstellung der akustischen Dateien für den Säugling oder das Kleinkind erfolgt mittels des erfindungsgemäßen Systems. Das System umfasst mindestens ein Speichermedium, auf welchem mindestens zwei unterschiedliche akustische Dateien gespeichert sind, die sich darin voneinander unterscheiden, dass Kinder mit normaler Sprachentwicklung das Hörerlebnis einer der mindestens zwei akustischen Dateien bevorzugen.

Dieses mindestens eine Speichermedium ist bevorzugt dem System entnehmbar. Die mindestens zwei akustischen Dateien können ausgetauscht werden. Austauschen bedeutet in diesem Zusammenhang, dass die mindestens zwei voneinander verschiedenen akustischen Dateien, durch mindestens zwei voneinander verschiedene akustische Dateien ersetzt werden. Auch diese akustischen Dateien sind so ausgewählt, das ein Kind mit normaler Sprachentwicklung das Hörerlebnis einer der mindestens zwei akustischen Dateien bevorzugt.

Erfindungswesentlich ist, dass dem Säugling oder dem Kleinkind ein direkter Zugriff auf Komponenten des erfindungsgemäßen Systems ermöglicht wird. Diese Komponenten sind die mindestens zwei Bedienelemente, die das selektive Abspielen der mindestens zwei akustischen Dateien auslösen. Weitere Komponenten des Systems sind dem Zugriff des Kindes entzogen, um eine Verletzungsgefahr beim Kind und eine Beeinträchtigung der Komponenten auszuschließen.

In einer bevorzugten Ausführungsform des erfindungsgemäßen Systems sind das mindestens eine Speichermedium zur Speicherung der akustischen Dateien, der mindestens eine Lautsprecher und das mindestens eine Detektionselement gemeinsam in einem Gehäuse, als separate Vorrichtung, aufgenommen. Das Gehäuse ist derart gestaltet, dass es einem Säugling oder Kleinkind nicht möglich ist, dieses zu öffnen und auch ein versehentliches Öffnen, beispielsweise beim Herunterfallen des Gehäuses, verhindert wird.

Erfindungsgemäß sind die Komponenten des Systems, mit denen der Säugling oder das Kleinkind in Kontakt kommen, kindgerecht und sicher für das Kind ausgestaltet. Kindgerecht bedeutet in diesem Zusammenhang unter anderem, dass diese Komponenten keine Teile enthalten, die das Kind entfernen und verschlucken könnte, die Vorrichtung nicht scharfkantig ist und aus einem Material besteht, das keine Schadstoffe an das Kind abgibt, auch wenn das Kind Komponenten des Systems in den Mund nimmt.
Die Komponenten des Systems werden so gewählt, dass auch bei längerem Gebrauch keine Hitze entsteht, bzw. das Kind keinen Zugriff auf warme Komponenten erhält.

In einer bevorzugten Ausführungsform ist die Vorrichtung zwischen 15 und 25 cm lang, 10 bis 20 cm breit und 7 bis 15 cm hoch. Mit diesen Abmessungen kann die Vorrichtung von Säuglingen und Kleinkindern gut ergriffen werden und auch beispielsweise im oder über dem Laufstall angebracht werden, ohne dass die Vorrichtung zu viel Raum einnimmt.

Weiterhin ist es erfindungsgemäß bevorzugt, dass die Vorrichtung ausreichend robust ist, um auch grobmotorischen Einwirkungen durch Säuglinge und Kleinkinder und einer dauerhaften Benutzung standzuhalten. Als dauerhafte Benutzung kann eine tägliche Benutzung über einen Zeitraum von mehreren Hundert Tagen angesehen werden.

Auch besteht das Gehäuse bevorzugt aus einem Material, das eine für Kinder angenehme Haptik besitzt, also beispielsweise aus Plastik oder Holz. Hierbei ist auch vorteilhaft, dass diese Materialien leicht zu reinigen sind. Insbesondere bevorzugt besitzt das Gehäuse keine scharfen Kanten.

In einer weiteren bevorzugten Ausführungsform ist das Gehäuse zumindest teilweise von einem lösbaren Bezug umschlossen, welcher jedoch den Zugriff auf die Bedienelemente nicht versperrt. Dieser Bezug ist vorzugsweise aus Materialien aufgebaut, die eine für das Kind angenehme Haptik besitzen. Das Material des Bezugs besitzt bevorzugt die haptischen Eigenschaften des Fells eines Plüschtiers. Der Bezug kann entfernt und gereinigt oder ersetzt werden. Bei dieser bevorzugten Ausführungsform kann das Material des Gehäuses beispielsweise auch aus Metall bestehen, weil der Bezug dieser Vorrichtung dem Gehäuse die für das Kind angenehmen haptischen Eigenschaften verleiht.

Um die Aufmerksamkeit des Kindes auf die Vorrichtung zu lenken, können das Gehäuse, die Bedienelemente und/oder der Bezug farbig oder bunt gestaltet sein.

Lediglich die mindestens zwei Bedienelemente können von dem Kind erreicht und auch betätigt werden, sind also nicht vollständig von einem Gehäuse umschlossen. Da vorgesehen ist, dass der Säugling oder das Kleinkind diese Bedienelemente bedient, müssen diese leicht, also mit geringem Kraftaufwand zu betätigen sein.

Durch das jeweilige Betätigen der mindestens zwei Bedienelemente wird das Abspielen jeweils einer akustischen Datei ausgelöst. Umfasst die Vorrichtung zwei Bedienelemente, so löst das Betätigen eines der Bedienelemente das Abspielen beispielsweise einer Sprachdatei mit natürlicher Sprachmelodie aus. Das Bedienen des anderen Bedienelements löst in diesem Ausführungsbeispiel das Abspielen einer Sprachdatei mit einer unnatürlichen Sprachmelodie aus.

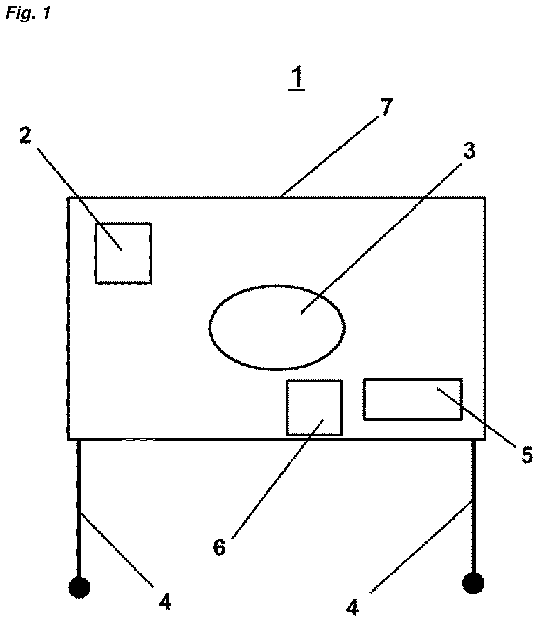

Figur 1 zeigt eine schematische Darstellung einer Ausführungsform der erfindungsgemäßen Vorrichtung 1, die in dieser Ausführungsform Teil des erfindungsgemäßen Systems ist. In dem Gehäuse 7 ist ein Speichermedium 2 zur Speicherung mindestens zweier unterschiedlicher akustischer Dateien angeordnet. Weiterhin sind Lautsprecher 3, Bedienelemente 4, Detektionselement 5 und Speichermedium 6 zur Speicherung der vom Detektionselement detektierten Daten, schematisch gezeigt.
Die Bedienelemente sind in dieser Darstellung als aus dem Gehäuse herausragende, langgestreckte Elemente gezeigt, die ein kugelförmiges Element aufweisen. Dieses kugelförmige Element dient dazu, dem Säugling oder Kind das Ergreifen der Bedienelemente zu erleichtern. Die langgestreckten Elemente können als Kordel, Schnur oder Stange ausgebildet sein.

Erfindungsgemäß bevorzugt umfasst das System ein Element zur Einstellung der Lautstärke beim Abspielen der akustischen Dateien, wobei dieses Element derart angeordnet ist, dass der Säugling oder das Kleinkind keinen Zugriff darauf haben. Bevorzugt ist dieses Element innerhalb des Gehäuses angeordnet.

Weiterhin bevorzugt ist eine Ausführungsform, bei welcher beim Betätigen der Bedienelemente ein optisches Signal erzeugt wird. Erfindungsgemäß ist dieses optische Signal ein Aufleuchten mindestens einer Lampe an der Vorrichtung. Besonders bevorzugt umfasst das System mindestens eine Lampe je Bedienelement und die optischen Signale werden bei Betätigung der Bedienelemente erzeugt. Vorteilhaft ist es, wenn die Lampen zum Ausgeben des optischen Signals in unmittelbarer Nähe der Bedienelemente oder auf diesen angeordnet sind.
Die mindestens eine Lampe ist erfindungsgemäß ausgewählt aus weißen oder farbigen Lampen, wobei die Lampen eine unterschiedliche oder gleiche Farbe besitzen können.

Weiterhin ist eine Vorrichtung erfindungsgemäß bevorzugt, welche mindestens ein Display umfasst, auf welchem die Anzahl der Betätigungen und/oder die Dauer der Betätigungen der mindestens zwei Bedienelemente angezeigt wird.

In einer weiteren bevorzugten Ausführungsform der Erfindung umfasst das System zwei Vorrichtungen, die jeweils separat in jeweils einem Gehäuse aufgenommen sind. Dieses System unterscheidet sich von dem oben beschriebenen System dadurch, dass jede der beiden Vorrichtungen nur ein Bedienelement umfasst und in jeder der beiden Vorrichtungen nur eine akustische Datei gespeichert ist. So ist in der einen Vorrichtung in einem Ausführungsbeispiel beispielsweise die Sprachdatei gespeichert, die eine natürliche Sprachmelodie aufweist, und in der zweiten Vorrichtung die Sprachdatei gespeichert ist, die eine unnatürliche Sprachmelodie aufweist. Die weiteren Merkmale entsprechen der oben beschriebenen Vorrichtung, die zwei Bedienelemente und zwei akustische Dateien umfasst.

In einer bevorzugten Ausführungsform bewirkt eine wiederholte Betätigung der Bedienelemente ein wiederholtes Abspielen der jeweiligen akustischen Datei.

Weiterhin bevorzugt ist eine Ausführungsform, bei welcher nach einer ersten Betätigung der Bedienelemente eine weitere Betätigung der Bedienelemente ein Pausieren des Abspielens bewirkt. Eine erneute Betätigung der Bedienelemente bewirkt eine Fortsetzung des Abspielens der akustischen Datei.

Bevorzugt ist auch eine Ausführungsform, bei welcher eine anhaltende Betätigung der Bedienelemente, also beispielsweise ein kontinuierliches Drücken eines Knopfes oder Halten eines Hebels, ein andauerndes Abspielen der jeweiligen akustischen Datei bewirkt. Ein Loslassen des Bedienelements bewirkt ein Beenden des Abspielens der akustischen Datei.

Je nachdem, welchen Hebel der Säugling oder das Kleinkind betätigt, wird in einem Ausführungsbeispiel die Sprachdatei mit natürlicher Sprachmelodie oder unnatürlicher Sprachmelodie abgespielt. Im erfindungsgemäßen Verfahren wird detektiert, wann, wie oft und/oder mit welcher Dauer die jeweiligen Bedienelemente betätigt werden. Die von der Detektionseinheit detektierten Daten, werden innerhalb der jeweiligen Vorrichtung auf einem Speicherelement gespeichert.

Die Speicherung der detektierten Daten kann bevorzugt mittels allgemein zugänglicher freier experimenteller Software (z.B. PsyScope, OpenSesame) erfolgen, aber auch mittels käuflich zu erwerbender Software (z.B. E-prime^{®} von PST - Psychology Software Tools), die speziell dafür programmiert wurde, behaviorale Reaktionsgeschwindigkeiten mit zeitlicher Präzision zu messen und zu speichern.

Befindet sich das mindestens eine Detektionselement innerhalb der jeweiligen Vorrichtung, werden die detektierten Daten bevorzugt auf einem Speicherelement innerhalb der Vorrichtung gespeichert. Dieses Speicherelement ist bevorzugt ausgewählt aus einer SD-Karte, einem USB-Speicher und einer Festplatte. In einer Ausführungsform der Erfindung wird das jeweilige Speicherelement zur Auswertung der detektierten Daten der Vorrichtung entnommen.

Alternativ erfolgt die Übertragung der detektierten Daten mittels Funkverbindung wie Bluetooth, oder mittels Kabel. In dieser Ausführungsform erfolgt die Speicherung der Daten außerhalb der Vorrichtung.

Um eine hohe Datensicherheit und Datenschutz zu gewährleisten, wird bei der am stärksten bevorzugten Ausführungsform des erfindungsgemäßen Systems, auf eine Datenübertragung mittels Funkverbindungen verzichtet.

In einer bevorzugten Ausführungsform der Erfindung erfolgt die Auswertung der detektierten Daten in der mindestens einen Vorrichtung.
Insbesondere werden bei dieser Ausführungsform die gespeicherten Daten auf einem Display angezeigt und von einer befugten Person abgelesen und notiert werden. Im Anschluss daran werden die gespeicherten Daten von der jeweiligen Vorrichtung gelöscht.

In einer Ausführungsform der Erfindung erfolgt die Auswertung innerhalb der Vorrichtung mittels eines Rechnersystems und einer Software. Als Software wird bevorzugt kommerzielle Software wie Microsoft^{®} Excel^{®}verwendet. Als Alternative kann beispielweise auch die Software SPSS^{®} von IBM^{®} oder experimentelle Software genutzt werden.

Die Stromversorgung der Vorrichtungen des Systems erfolgt mittels Akkus oder Batterien, so dass keine Kabel zur Stromversorgung dauerhaft an den Vorrichtungen angebracht sind. Die Betriebsdauer beträgt mindestens 24 Stunden und die Standby-Dauer (ohne Betätigung der Bedienelemente) beträgt mehr als 240 Stunden.

In einer bevorzugten Ausführungsform erfolgt das Aufladen des Akkus mittels eines externen Ladegeräts, wobei die Steckerbuchse an den Vorrichtungen mit einem Verschlusselement verschlossen ist, das bevorzugt nicht von einem Säugling oder Kleinkind entfernt werden kann. In einer bevorzugten Ausführungsform ist das Verschlusselement eine Kappe.

In einer weiteren Ausführungsform der Erfindung wird der Ladezustand der Batterien bzw. des Akkus angezeigt.

Erfindungsgemäß ist vorgesehen, dass die oben beschriebenen Vorrichtungen, also die Zwei-Bedienelement-Vorrichtung oder die zwei Ein-Bedienelement-Vorrichtungen beispielsweise an einem Kinderbett, einem Laufstall oder über dem Kinderbett oder Laufstall angeordnet sind. Die Anordnung erfolgt derart, dass der Säugling oder das Kleinkind die Vorrichtungen sehen kann und die Bedienelemente erreichen kann. In einem weiteren bevorzugten Ausführungsbeispiel des erfindungsgemäßen Verfahrens werden die eine Zwei-Bedienelement-Vorrichtung oder die zwei Ein-Bedienelement-Vorrichtungen in dem Aufenthaltsbereich des Säuglings oder Kleinkinds abgelegt.

Das Kind betätigt die Bedienelemente, wobei eines der Bedienelemente das Abspielen einer der mindestens zwei verschiedenen akustischen Dateien auslöst, während das andere Bedienelement das Abspielen einer davon verschiedenen akustischen Datei auslöst.

In einem bevorzugten Ausführungsbeispiel sind die akustischen Dateien Sprachdateien, die sich in der Sprachmelodie, natürlich und unnatürlich, unterscheiden. Durch das Betätigen eines der Bedienelemente hat das Kind das Hörerlebnis einer Sprachdatei mit natürlicher Sprachmelodie. Betätigt das Kind das andere Bedienelement, hat das Kind das Hörerlebnis einer Sprachdatei mit unnatürlicher Sprachmelodie.

Es wird detektiert, wie oft und wie lange das Kind die jeweiligen Bedienelemente betätigt. Diese Werte werden gesammelt ausgewertet, so dass festgestellt werden kann, ob das Kind eine bestimmte Sprachdatei bevorzugt abgespielt hat. Wenn sich ein Baby typisch entwickelt, sollte es das Bedienelement häufiger betätigen, welches die Sprachdatei mit einer natürlichen Sprachmelodie abspielt.

Eine solche Vorliebe für die Sprachdatei mit einer natürlichen Sprachmelodie wird jedoch nicht von Kindern erwartet, die später eine Sprachentwicklungsstörung entwickeln.
Somit könnte man den Säugling oder das Kleinkind der Risikogruppe für das Bestehen einer Sprachentwicklungsstörung zuordnen, wenn keine oder nur eine schwache Präferenz für eine akustischen Datei zu erkennen ist, oder eine Präferenz für eine nicht natürliche akustische Datei vorliegt.

Das erfindungsgemäße Verfahren kann bereits bei Kindern im Alter von 9 bis 12 Monaten angewendet werden, da Kinder in diesem Alter bereits den spielerischen Trieb und die körperlichen Fähigkeiten für den Umgang mit den Vorrichtungen besitzen.

### Bezugszeichenliste

- 1 -: erfindungsgemäße Vorrichtung (Teil des erfindungsgemäßen Systems)
- 2 -: Speichermedium für akustische Dateien
- 3 -: Lautsprecher
- 4 -: Bedienelement
- 5 -: Detektionselement
- 6 -: Speicherelement für detektierte Daten
- 7 -: Gehäuse

## Patentansprüche

1. System zum Erfassen von Daten zur Bevorzugung eines Hörerlebnisses, umfassend:
mindestens ein Speichermedium zur Speicherung von Dateien, auf welchem mindestens zwei unterschiedliche akustische Dateien gespeichert sind, die sich darin voneinander unterscheiden, dass Kinder mit normaler Sprachentwicklung das Hörerlebnis einer der mindestens zwei akustischen Dateien bevorzugen,
mindestens einen Lautsprecher, zur Wiedergabe der akustischen Dateien,
mindestens zwei Bedienelemente, die selektiv das Abspielen der mindestens zwei akustischen Dateien initiieren,
mindestens ein Detektionselement, welches die Anzahl und/oder Dauer der Betätigungen der mindestens zwei Bedienelemente detektiert,
und mindestens ein Speicherelement, welches die vom mindestens einen Detektionselement detektierten Daten speichert.

2. System, gemäß Anspruch 1, **dadurch gekennzeichnet, dass** die mindestens zwei verschiedenen akustischen Dateien Sprachdateien oder nichtsprachliche Ton-Dateien sind, die ausgewählt sind aus Sprachdateien mit unterschiedlichem Betonungsmuster, Sprachdateien mit unterschiedlichem Satzintonationsmuster, Sprachdateien mit unterschiedlichem Sprachrhythmus, Sprachdateien mit unterschiedlichen Sprachlauten, Sprachdateien mit unterschiedlichen Phonemen, Sprachdateien mit für das Kind muttersprachlicher oder nicht muttersprachlicher Sprache, Sprachdateien mit für das Kind nicht muttersprachlicher Sprache, Sprachdateien mit kindgerechter Sprache, Sprachdateien mit nicht kindgerechter Sprache, Geräuschen, nichtsprachlichen Tonabfolgen und Melodien.

3. System, gemäß Anspruch 1 oder 2, **dadurch gekennzeichnet, dass** das System mindestens eine Rechnereinheit mit Software umfasst.

4. System, gemäß Anspruch 3, **dadurch gekennzeichnet, dass** die mindestens eine Rechnereinheit mit Software und/oder das mindestens eine Speicherelement Komponenten des Detektionselements sind.

5. System, gemäß einem der vorangehenden Ansprüche, **dadurch gekennzeichnet, dass** das System mindestens eine Vorrichtung umfasst, in welcher Elemente des Systems gemeinsam in einem Gehäuse aufgenommen sind.

6. System, gemäß Anspruch 5, **dadurch gekennzeichnet, dass** das System eine Vorrichtungen umfasst, in welcher ein Speichermedium, zur Speicherung mindestens einer akustischen Datei, ein Lautsprecher und ein Detektionselement gemeinsam in einem Gehäuse aufgenommen sind, an welchem zwei Bedienelemente angeordnet sind.

7. System, gemäß Anspruch 5, **dadurch gekennzeichnet, dass** das System zwei separate Vorrichtungen umfasst, in welchen jeweils ein Speichermedium, zur Speicherung mindestens einer akustischen Datei, ein Lautsprecher und ein Detektionselement gemeinsam in einem Gehäuse aufgenommen sind und an jedem der beiden Gehäuse ein Bedienelement angeordnet ist.

8. System, gemäß einem der Ansprüche 5 bis 7, **dadurch gekennzeichnet, dass** die mindestens eine Vorrichtung jeweils mindestens eine Rechnereinheit mit Software umfasst.

9. System, gemäß einem der vorangehenden Ansprüche, **dadurch gekennzeichnet, dass** die mindestens zwei akustischen Dateien Sprachdateien sind, die mit unterschiedlicher Sprachmelodie gesprochener identischer Text sind, und/oder die mindestens zwei Sprachdateien von demselben Sprecher gesprochen wurden.

10. System, gemäß einem der vorangehenden Ansprüche, **dadurch gekennzeichnet, dass**
das mindestens eine Speichermedium zur Speicherung von Sprachdateien ein USB-Stick, eine SD-Speicherkarte oder eine Festplatte ist, und/oder
die mindestens zwei Bedienelemente ausgewählt sind aus mechanischen Knöpfen, mechanischen Hebeln, mechanischen Tasten und Berührungssensoren; und/oder
das mindestens eine Detektionselement ausgewählt ist aus mechanischen, elektronischen und softwaregestützten Detektionselementen, und/oder
das mindestens eine Detektionselement ein Speicherelement umfasst, welches ein USB-Stick, eine SD-Speicherkarte oder eine Festplatte ist.

11. Verfahren zum Erfassen von Daten zur Bevorzugung eines Hörerlebnisses bei Säuglingen und Kleinkindern,
a) wobei man ein System gemäß einem der Ansprüche 1 bis 10 bereitstellt,
b) man dem Säugling oder Kleinkind mindestens einen Zeitraum lang den Zugriff auf die Bedienelemente des Systems ermöglicht,
c) man detektiert, wie oft und/oder wie lange der Säugling oder das Kleinkind die mindestens zwei Bedienelemente des Systems in dem mindestens einen Zeitraum betätigt,
d) man basierend auf der Anzahl und/oder Dauer der Betätigungen der mindestens zwei Bedienelemente des Systems beim Säugling oder Kleinkind eine Bevorzugung einer der akustischen Dateien ermittelt.

12. Verfahren, gemäß Anspruch 11, **dadurch gekennzeichnet, dass** man das Verfahren im natürlichen Lebensumfeld des Säuglings oder Kleinkinds durchführt.

13. Verfahren, gemäß einem der Ansprüche 11 oder 12, **dadurch gekennzeichnet, dass** der mindestens eine Zeitraum in Schritt b) zwischen 1 und 20 Minuten beträgt.

14. Verfahren, gemäß einem der Ansprüche 11 bis 13, **dadurch gekennzeichnet, dass** man Schritt b) über mehrere Zeiträume durchführt.
